(19) Europäisches Patentamt
European Patent Office
Office européen des brevets

(11) Veröffentlichungsnummer: **0 025 501**
**B1**

(12) # EUROPÄISCHE PATENTSCHRIFT

(45) Veröffentlichungstag der Patentschrift: **29.12.82**

(51) Int. Cl.³: **C 07 D 209/42**, A 61 K 31/40

(21) Anmeldenummer: **80104465.2**

(22) Anmeldetag: **29.07.80**

(54) **Neue N-Aminoalkylindol-Derivate und ihre Salze; Verfahren zu deren Herstellung und diese Verbindungen enthaltende Arzneimittel.**

(30) Priorität: **02.08.79 DE 2931323**

(43) Veröffentlichungstag der Anmeldung: **25.03.81 Patentblatt 81/12**

(45) Bekanntmachung des Hinweises auf die Patenterteilung: **29.12.82 Patentblatt 82/52**

(84) Benannte Vertragsstaaten: **AT BE CH DE FR GB IT LI LU NL SE**

(56) Entgegenhaltungen: **DE-A-2 727 047** **GB-A-959 203**

(73) Patentinhaber: **Kali-Chemie Pharma GmbH, Hans-Böckler-Allee 20 Postfach 220, D-3000 Hannover 1 (DE)**

(72) Erfinder: **Heinemann, Henning Dipl.-Chem. Dr. rer. nat., Bergiusstrasse 18, D-3000 Hannover 51 (DE)**
Erfinder: **Ohlendorf, Heinrich-Wilhelm Dipl.-Chem. Dr., Lilienstrasse 19, D-3000 Hannover 1 (DE)**
Erfinder: **Wolf, Klaus-Ullrich, Dr., Imkersweg 6, D-3165 Hänigsen (DE)**

(74) Vertreter: **Lauer, Dieter, Dr. et al, c/o Kali-Chemie Aktiengesellschaft Postfach 220, D-3000 Hannover 1 (DE)**

**EP 0 025 501 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue N-Aminoalkylindol-Derivate und ihre Salze; Verfahren zu deren Herstellung und diese Verbindungen enthaltende Arzneimittel

Die Erfindung betrifft neue N-Aminoalkylindol-Derivate, deren pharmakologisch verträgliche Salze, Verfahren zu deren Herstellung sowie diese Verbindungen enthaltende pharmazeutische Zusammensetzungen.

Der Erfindung liegt die Aufgabe zugrunde, neue N-Aminoalkylindol-Derivate mit wertvollen pharmakologischen und therapeutischen Eigenschaften zu schaffen.

Überraschenderweise wurde nun gefunden, daß die neuen Verbindungen eine gute Wirkung auf die Motilität des Magens haben.

Gegenstand der Erfindung sind daher neue N-Aminoalkylindol-Derivate der Formel I

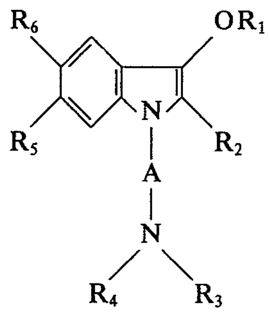

worin $R_1$ ein Wasserstoffatom, eine gegebenenfalls mit einem Phenylrest substituierte Alkylgruppe mit 1 bis 4 Kohlenstoffatomen oder ein Acetylrest und $R_2$ die Hydroxycarbonylgruppe, eine Alkoxycarbonylgruppe mit 1 bis 4 Kohlenstoffatomen, die Cyanogruppe oder eine Amino-, Monoalkylamino- oder Dialkylaminocarbonylgruppe mit Alkylresten mit 1 bis 4 Kohlenstoffatomen bedeuten, wobei, wenn $R_2$ die Hydroxycarbonylgruppe ist, $R_1$ kein Wasserstoffatom bedeutet, A eine $C_2-C_5$-Alkylengruppe ist, $R_3$ und $R_4$ gleich oder verschieden sind und Wasserstoffatome oder Alkylreste mit 1 bis 4 Kohlenstoffatomen bedeuten, oder $R_3$ und $R_4$ gemeinsam mit dem Stickstoffatom, an das sie gebunden sind, eine cyclische Gruppe mit 5 bis 7 Kettengliedern bilden, $R_5$ und $R_6$ gleich oder verschieden sind und Wasserstoffatome, Halogenatome oder Alkyl- oder Alkoxygruppen mit je 1 bis 3 Kohlenstoffatomen bedeuten oder einer der beiden Reste $R_5$ und $R_6$ eine Trifluormethyl- oder Nitrogruppe und der andere ein Wasserstoffatom bedeutet, sowie deren Säureadditionssalze.

Als Alkylreste für $R_1$, $R_3$ und $R_4$ kommen Alkylreste mit 1 bis 4 Kohlenstoffatomen, welche geradkettig oder verzweigt sind, in Frage, so Methyl, Äthyl, Propyl, Isopropyl, n-Butyl, 1-Methylpropyl, 2-Methylpropyl und tert.-Butyl. Besonders bevorzugt werden Methyl, Äthyl, Propyl, Isopropyl und tert.-Butyl.

Bedeutet $R_1$ einen mit Phenyl substituierten Alkylrest, so sind Benzyl und Phenäthyl bevorzugt. Als Alkylreste der Mono- oder Dialkylaminocarbonylgruppen für $R_2$ eignen sich die oben genannten Reste, vor allem Methyl, Äthyl, Propyl und Isopropyl.

A bedeutet einen geradkettigen oder verzweigten $C_2-C_5$-Alkylenrest, wobei neben Methylen, Äthylen, Propylen, Isopropylen, n-Butylen, 1-Methylpropylen und 2-Methylpropylen noch Pentylen, Neopentylen oder Isopentylen möglich sind.

Als Halogenatome für $R_5$ und $R_6$ kommen Fluor, Chlor, Brom und Jod in Frage, insbesondere Chlor und Brom. Im Falle der Alkyl- oder Alkoxysubstitution für $R_5$ und $R_6$ eignen sich die Alkylreste Methyl, Äthyl, Propyl und Isopropyl, wobei insbesondere bei Disubstitution der Methylrest bevorzugt ist.

Die cyclische Gruppe $NR_3R_4$ kann die Bedeutung Pyrrolidino, Piperidino oder Azacycloheptyl haben. Als pharmakologisch verträgliche Säureadditionssalze der Verbindungen der Formel I kommen die Hydrochloride, Hydrobromide, Hydrogensulfate, Nitrate, Phosphate, aber auch Salze mit organischen Säuren, wie Cyclohexylaminosulfonsäure, Maleinsäure, Toluol-4-sulfonsäure oder Amidosulfonsäure, in Frage.

Die neuen Verbindungen und deren Säureadditionssalze werden dadurch erhalten, daß man ein Alkalimetallsalz des Indols der Formel II

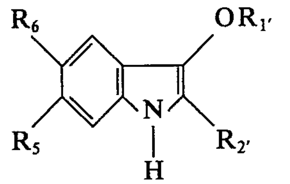

worin $R_5$ und $R_6$ die obige Bedeutung haben, $R_{1'}$ eine gegebenenfalls mit einem Phenylrest

substituierte Alkylgruppe oder eine Acetylgruppe und $R_2$, eine Alkoxycarbonylgruppe der obigen Bedeutung oder eine Cyanogruppe sind, in einem inerten Lösungsmittel

a) nacheinander mit einem Dihalogenalkan der Formel III

$X_1-A-X_2$

worin A die obengenannte Bedeutung hat, $X_1$ und $X_2$ gleich oder verschieden sein können und Halogen, vorzugsweise Chlor oder Brom, bedeuten, und einem Amin der Formel IV

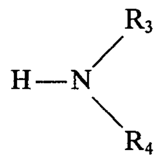

worin $R_3$ und $R_4$ die obengenannte Bedeutung haben, oder allein mit einem Halogenalkylamin der Formel V

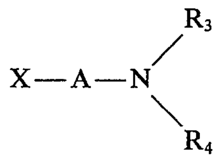

worin A die obengenannte Bedeutung besitzt, $R_3$ und $R_4$ die obengenannten Bedeutungen außer Wasserstoffatom haben und X ein Halogenatom, vorzugsweise Chlor oder Brom, ist, umsetzt zu Verbindungen der Formel I

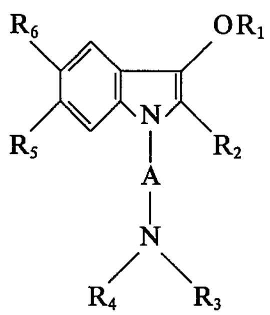

worin A, $R_3$, $R_4$, $R_5$ und $R_6$ die obigen Bedeutungen haben, und $R_1$ eine gegebenenfalls mit einem Phenylrest substituierte Alkylgruppe oder einer Acetylgruppe und $R_2$ eine Cyano- oder Alkoxycarbonylgruppe sind,

b) gewünschtenfalls eine Cyano- oder Alkoxycarbonylgruppe $R_2$ in die Hydroxycarbonylgruppe $R_2$ überführt,

c) gewünschtenfalls eine Cyano-, Alkoxycarbonyl- oder Hydroxycarbonylgruppe $R_2$ in eine gegebenenfalls mono- oder disubstituierte Aminocarbonylgruppe $R_2$ überführt,

d) gewünschtenfalls in den nach a) oder c) erhaltenen Verbindungen, worin $R_1$ Acetyl bedeutet, die Acetylgruppe $R_1$ hydrolytisch abspaltet und die 3-Hydroxyverbindungen isoliert,

e) gewünschtenfalls in den nach a) oder c) erhaltenen Verbindungen, worin $R_1$ Benzyl bedeutet, die Benzyloxygruppe hydrogenolytisch spaltet und die 3-Hydroxyverbindungen isoliert,

f) gewünschtenfalls in den nach a) oder c) erhaltenen Verbindungen, worin $R_1$ Alkyl bedeutet, die Alkoxygruppe hydrolytisch spaltet und die 3-Hydroxyverbindungen isoliert und

g) die so erhaltenen Basen isoliert und diese gewünschtenfalls in die entsprechenden Säureadditionssalze überführt.

Die als Ausgangsmaterialien dienenden Indole der Formel II sind bekannt. Sie können z. B. durch basische Cyclisierung des entsprechenden N-Alkoxycarbonylmethylanthranilsäureesters und anschließende Verätherung des gebildeten Enolates dargestellt werden (s. z. B. FR-PS 1 503 908, in welcher diese Indole als Zwischenprodukte beschrieben sind).

Die 3-Hydroxyindol-Derivate der Formel II werden vor ihrer Umsetzung mit einer Schutzgruppe versehen, d. h. vorzugsweise als 3-Acetoxyverbindungen eingesetzt.

Das vorstehend unter a) beschriebene Verfahren zur Einführung der Alkylamingruppe stellt eine an sich bekannte Alkylierung am Stickstoffatom in 1-Stellung des Indols dar. Vorteilhafterweise wird

zunächst eine Lösung eines Alkalimetallsalzes des Indols der Formel II erzeugt. Zweckmäßigerweise führt man die Umsetzung in einem inerten Lösungsmittel, wie Dimethylformamid, Sulfolan, 1,4-Dioxan, Dimethylsulfoxid oder Toluol, zwischen −20°C und Raumtemperatur, vorzugsweise zwischen −10 und 10°C durch. Als Basen benutzt man Alkalimetallalkoholate, wie Natriummethylat, Natriumäthylat oder Kalium-tert.-butylat, Alkalimetallamide, wie Natriumamid oder Lithiumdiisopropylamid, oder Alkalimetallhydride wie Natriumhydrid.

Die Alkylamineinführung kann dadurch erfolgen, daß man die obige Lösung entweder nacheinander mit einem Dihalogenalkan und einem Amin oder direkt mit einem Halogenalkylamin umsetzt.

Im ersten Fall wird das Dihalogenalkan der Formel III zugegeben und vorzugsweise auf eine Temperatur zwischen 60 und 90°C bei Normaldruck oder erhöhtem Druck erhitzt.

Unter diesen Reaktionsbedingungen bildet sich das N-Halogenalkyl)-indol-Derivat

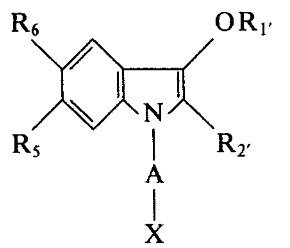

worin $R_{1'}$, $R_{2'}$, $R_5$, $R_6$ und A die obige Bedeutung haben und X Halogen, vorzugsweise Chlor oder Brom, bedeutet. Bei Einsatz eines Dihalogenalkans III mit verschiedener Substitution bedeutet X das reaktionsträge Halogenatom, vorzugsweise Chlor.

Vor der anschließenden Umsetzung mit Ammoniak oder Alkylaminen kann man das N-(Halogenalkyl)-indol-Derivat aus dem Reaktionsgemisch isolieren und gegebenenfalls umkristallisieren. Die Reaktion mit dem Amin der Formel IV kann in einem inerten Lösungsmittel wie Dimethylformamid, Sulfolan, Tetrahydrofuran, 1,4-Dioxan, Toluol, Dimethylsulfoxid oder Gemischen derselben, bei Normaldruck oder erhöhtem Druck durchgeführt werden. Die Temperaturen liegen zwischen Raumtemperatur und der Siedetemperatur des Lösungsmittels, vorzugsweise zwischen 40 und 60°C. Als Säurebinder können Alkali- oder Erdalkalicarbonate, wie Natriumcarbonat, Kaliumcarbonat oder Calciumcarbonat dienen, außerdem tertiäre Amine, wie Triäthylamin oder Pyridin, aber auch die im Überschuß verwendeten Amine der Formel IV. Letztere können, im Überschuß eingesetzt, auch als Lösungsmittel dienen.

Im zweiten Fall wird zu der Lösung des Alkalisalzes des Indolderivates der Formel II das Halogenalkylamin der Formel V zugegeben und bei Temperaturen zwischen Raumtemperatur und dem Siedepunkt des Lösungsmittels, vorzugsweise zwischen 60 und 90°C, bei Normaldruck oder erhöhtem Druck die Umsetzung durchgeführt.

Zur Herstellung der N-Aminoalkylindole der Formel I, in welchen $R_2$ eine Hydroxycarbonylgruppe ist, kann man die Verbindungen der Formel I, in welchen $R_2$ eine Cyano- oder eine Alkoxycarbonylgruppe ist, hydrolytisch spalten. Bevorzugt wird man die Ester in an sich bekannter Weise im sauren oder alkalischen Medium hydrolysieren. Zur sauren Hydrolyse eignen sich verdünnte Salzsäure oder Schwefelsäure bei Temperaturn von 60 bis 100°C. Geeignete Lösungsmittel sind niedere Alkohole, Tetrahydrofuran, 1,4-Dioxan, allein oder im Gemisch mit Wasser. Die basische Hydrolyse geschieht vorzugsweise mit wäßrigem Alkali, z. B. Natriumhydroxid, Kaliumhydroxid, Kaliumcarbonat oder Natriumcarbonat, bei einer Temperatur zwischen 15 und 100°C, bevorzugt zwischen 50 und 70°C, und liefert das entsprechende Alkalisalz der Carbonsäure, welches durch Ansäuern mit einer Mineralsäure, z. B. Salzsäure oder Schwefelsäure, oder auch einer organischen Säure wie Essigsäure, in die freie Carbonsäure I überführt werden kann.

Zur Herstellung der N-Aminoalkylindole der Formel I, in welchen $R_2$ eine gegebenenfalls substituierte Aminocarbonylgruppe ist, kann man von den Verbindungen der Formel I ausgehen, in welchen $R_2$ eine Cyano-, Alkoxycarbonyl- oder Hydroxycarbonylgruppe ist. Die Cyanoverbindungen kann man z. B. durch Umsetzung in Mineralsäuren oder alkalisch, z. B. mit Natrium- oder Kaliumhydroxid, gegebenenfalls unter Zugabe von Wasserstoffperoxid, in einem geeigneten Lösungsmittel in die Amide der Formel I überführen. Die Alkoxycarbonylverbindungen der Formel I lassen sich durch Ammonolyse oder Aminolyse in an sich bekannter Weise durch Behandlung mit Ammoniak oder einem primären bzw. sekundären Amin unter Normaldruck oder erhöhtem Druck in die entsprechenden Amide überführen.

Besonders vorteilhaft ist die Umwandlung der Hydroxycarbonylgruppe $R_2$ in die gegebenenfalls substituierte Amidgruppe. Die Umwandlung einer Säurefunktion in eine Amidfunktion kann in an sich bekannter Weise direkt durch Umsetzung der freien Säure mit Ammoniak oder primären oder sekundären Alkylaminen bewirkt werden.

Es kann von Vorteil sein, diese Umwandlung zweistufig durchzuführen, indem man die Säure vorerst in ein zur Darstellung von Säureamiden geeignetes reaktives Derivat, z. B. in ein Säurehalogenid, insbesondere das Säurechlorid, in ein reaktionsfähiges Säureanhydrid, in ein Säureimidazolid oder

auch in ein Kondensationsprodukt mit N,N'-Dicyclohexylcarbodiimid überführt, worauf das Säurederivat anschließend in situ mit Ammoniak oder dem Alkylamin umgesetzt wird.

Zur Herstellung des Säureanhydrids setzt man die Säure zweckmäßigerweise mit einem Chlorameisensäurealkylester, z. B. dem Äthylester, um. Die Reaktionstemperatur liegt beispielsweise zwischen −5°C und Raumtemperatur. Als Reaktionsmedium kann ein inertes Lösungsmittel dienen, z. B. Chloroform, Toluol, Tetrahydrofuran oder Dimethylformamid. Die Überführung in ein Imidazolid geschieht z. B. mit Carbonyldiimidazol in einem inerten Lösungsmittel wie Tetrahydrofuran oder Chloroform bei Temperaturen zwischen −10°C und Raumtemperatur; bei ähnlichen Temperaturen kann auch die Kondensation der Säure mit N,N'-Dicyclohexylcarbodiimid in einem inerten organischen Lösungsmittel, wie Methylenchlorid, 1,4-Dioxan, Toluol oder Gemischen derselben, durchgeführt werden.

Die Umsetzung der oben beschriebenen Säurederivate mit Ammoniak oder einem primären oder sekundären Alkylamin erfolgt vorteilhafterweise bei einer Temperatur zwischen −5°C und Raumtemperatur in einem Lösungsmittel, wie Wasser, Chloroform, Toluol, Tetrahydrofuran, Dimethylformamid und Gemischen derselben. Es kann vorteilhaft sein, das zur Umsetzung benutzte Amin im Überschuß als säurebindendes Reagenz zu verwenden oder die Umsetzung in Gegenwart anderer säurebindender Reagenzien, wie z. B. Kaliumcarbonat, Natriumcarbonat, Natriumhydroxid, Kaliumhydroxid, Triäthylamin oder Pyridin, durchzuführen. Im Überschuß eingesetzt, können die Amine zusätzlich als Lösungsmittel dienen.

Die Verbindungen der Formel I, in welchen $R_1$ ein Wasserstoffatom und $R_2$ eine Cyano-, Alkoxycarbonyl- oder eine gegebenenfalls substituierte Aminocarbonylgruppe sind und $R_3$, $R_4$, $R_5$ und $R_6$ die obige Bedeutung haben, kann man aus den nach a) oder c) erhaltenen Verbindungen dadurch erhalten, daß man die als Schutzgruppe dienende Acetylgruppe abspaltet. Dazu wird vorzugsweise das Enolacetat der Formel I in einem Lösungsmittel, wie Wasser, Methanol, Äthanol, Aceton, Tetrahydrofuran, 1,4-Dioxan oder Gemischen derselben, mit einem Alkali- oder Erdalkalimetallcarbonat, wie Natriumcarbonat, Kaliumcarbonat, oder auch mit Ammoniumcarbonat, bei Temperaturen zwischen 0 und 40°C umgesetzt.

Ebenso kann durch Hydrierung des entsprechenden Benzylenoläthers der Formel I in einem inerten organischen Lösungsmittel, wie Methanol, Äthanol, Äthylacetat oder 1,4-Dioxan, gegebenenfalls unter Zuhilfenahme eines Katalysators wie z. B. Palladium/Kohle, bei Normaldruck oder erhöhtem Druck und Temperaturen zwischen Raumtemperatur und 150°C, vorzugsweise zwischen 40 und 80°C, die Darstellung der 3-Hydroxyderivate der Indole der Formel I erfolgen.

Die Darstellung des Enols kann aber auch durch Umsetzung der entsprechenden 3-Alkoxyindole mit einer nicht oxydierenden Mineralsäure in einem organischen Lösungsmittel, z. B. mit konzentrierter Chlorwasserstoffsäure in Methanol, Äthanol oder 1,4-Dioxan, bei Temperaturen, die in der Nähe des Siedepunktes liegen, oder aber durch Kochen unter Rückfluß, stattfinden.

Die neuen Verbindungen und ihre Salze weisen, soweit sie nicht wichtige Zwischenprodukte sind, wertvolle therapeutische Eigenschaften auf, vor allem zeigen sie eine ausgeprägte Wirkung bei Motilitätsstörungen im Magen-Darm-Kanal.

Es ist bekannt, daß einem großen Teil der gastroenterologischen Beschwerden funktionelle Störungen zugrundeliegen. In zunehmendem Maße werden Motilitätsstörungen, insbesondere des Magens und seiner Sphincteren, als Ursache verschiedener gastro-intestinaler Erkrankungen erkannt. [s. Leber, Magen, Darm, 8 (1978) Nr. 4, S. 177−182 und 184−190, bzw. Internist 20 (1979) S. 10−17]. Vor allem eine Pylorusinkompetenz, die für den duodenogastrischen Reflux verantwortlich gemacht wird, nimmt breiten Raum in der Diskussion um die pathophysiologischen Ursachen verschiedener Störungen ein [Am.J. Dig. Dis., 21 (197?) Nr. 2, S. 165−173]. Demnach werden die Refluxgastritis, das Ulcus ventriculi und Ulcus duodeni sowie Völlegefühl, Übelkeit und epigastrische Schmerzen ohne anatomisch faßbare Ursache durch Störungen der Magenpassage hervorgerufen oder in ihrem Verlauf kompliziert.

Ziel einer hier ansetzenden Therapie ist daher die Wiederherstellung der physiologischen Magenmotilität und der ungestörten Magenpassage.

Überraschenderweise wurde gefunden, daß die erfindungsgemäßen Substanzen einen solchen Effekt besitzen. Im Tierexperiment werden unter ihrem Einfluß die peristaltischen Wellen des Magens verstärkt, wobei die Frequenz der Bewegungen zugunsten kräftiger, tiefdurchschnürender Wellen abnimmt. Aus diesen Wirkungen kann man auf eine Verbesserung der Magenentleerung schließen.

## Beschreibung der pharmakologischen Untersuchungsmethoden

### 1. Akute Toxizität

Die akute 7-Tage-Toxizität wird nach einmaliger Applikation intraperitoneal an der weißen nüchternen NMRI-Maus bestimmt. Die Berechnung der $LD_{50}$-Werte erfolgt über EDV durch eine Probitanalyse [L. Cavalli-Sforza, Gustav-Fischer-Verlag, Stuttgart (1964), Grundbegriffe der Biometrie, S. 153 ff.].

2. Prüfung der Magenperistaltik

Zur Bestimmung der Magenperistaltik werden etwa 200 g schweren, mit Ketamino-Hydrochlorid/Xylazin narkotisierten Ratten in die Vena jugularis ein Gefäß-Katheter und in die Trachea ein Trachealkatheter eingeführt. In den Magen wird eine Magensonde eingebunden, die über einen Dreiweghahn mit einem Statham-Druckgeber (P 23 DB) verbunden wird. Der Magen wird am Pylorus und an der Cardia durch eine Ligatur verschlossen. Der Magen wird mit 3 ml 0,9%iger wäßriger NaCl-Lösung gefüllt. Die vom Magen erzeugten Druckwellen werden kontinuierlich von einem Watanabe-Multicorder (MC 641) registriert. Zur Bestimmung der Wirkung der Testsubstanzen werden diese in physiologischer Natriumchloridlösung gelöst oder in Tylose MH 50 suspendiert in einer Dosis von 20 mg/kg intraperitoneal appliziert. Verglichen werden die vor und nach Gabe der Substanzen auftretenden Amplituden und Frequenzen der Druckwellen des Magens.

Die Auswertung ergibt, daß kurz nach Applikation der erfindungsgemäßen Substanzen eine beträchtliche Amplitudensteigerung auftritt. Dieser Effekt in Verbindung mit einer unterschiedlich ausgeprägten Frequenzsenkung führt zu einer verbesserten Magenpassage.

Nach den beschriebenen Methoden wurden beispielsweise folgende Substanzen untersucht:

A 1-(β-Dimethylaminäthyl)-2-methoxycarbonyl-3-methoxy-indol-hydrochlorid
B 1-(β-Dimethylaminoäthyl)-2-methoxycarbonyl-3-methoxy-6-methyl-indol-hydrochlorid
C 1-(β-Dimethylaminoäthyl)-2-methoxycarbonyl-3-äthoxy-5-chlor-indol-hydrochlorid
D 1-(β-Dimethylaminoäthyl)-2-methoxycarbonyl-3-isopropyloxy-5-methyl-indol-hydrochlorid
E 1-(β-Dimethylaminoäthyl)-2-methoxycarbonyl-3-benzyloxy-5-chlor-indol-toluol-4-sulfonat
F 1-(β-Dimethylaminoäthyl)-2-äthoxycarbonyl-3-äthoxy-6-chlor-indol-toluol-4-sulfonat
G 1-(γ-Isopropylaminopropyl)-2-methoxycarbonyl-3-methoxy-indol-hydrochlorid
H 1-(δ-Dimethylaminobutyl)-2-methoxycarbonyl-3-methoxy-indol-toluol-4-sulfonat
I 1-(γ-N-Piperidinopropyl)-2-methoxycarbonyl-3-methoxy-indol-maleinat
K 1-(β-Dimethylaminoäthyl)-2-hydroxycarbonyl-3-methoxy-indol
L 1-(β-Dimethylaminoäthyl)-2-aminocarbonyl-3-methoxy-indol
M 1-(β-Dimethylaminoäthyl)-2-methylaminocarbonyl-3-methoxy-indol

Tabelle
Magendruckmessung

| Substanz | Faktor der Amplitudensteigerung | Frequenzsenkung in % | $LD_{50}$ i.p. mg/kg |
|---|---|---|---|
| A | 6,2 | 3,3 | 171 |
| B | 22,7 | 7,0 | 68 |
| C | 1,4 | 9,0 | 141 |
| D | 6,9 | 19,0 | 137 |
| E | 0,6 | − 2,0 | 283 |
| F | 10,9 | 5,0 | 226 |
| G | 8,3 | 29,0 | 91 |
| H | 3,5 | 11,0 | 164 |
| I | 8,6 | 15,0 | 141 |
| K | 3,6 | 21,0 | 967 |
| L | 13,9 | 28,0 | 68 |
| M | 21,4 | 13,0 | 68 |

Die vorhergehende Tabelle enthält die gemessenen Werte. Aus ihnen geht eindeutig hervor, daß schon geringe Dosen der erfindungsgemäßen Substanzen und deren Säureadditionssalze eine signifikante Verstärkung der peristaltischen Wellen des Magens bewirken, wobei die hohe Wirksamkeit und die geringe Toxizität der Substanzen auf eine gute Verträglichkeit derselben hinweisen. Ein weiterer Vorteil ist der beobachtete rasche Wirkungseintritt.

Die pharmakologisch beobachteten Wirkungen lassen darauf schließen, daß die erfindungsgemäßen Substanzen am Menschen Störungen der Magen-Darmfunktion, wie beispielsweise Pylorusstenosen, duodenogastrischen Reflux sowie atonische Zustände, beheben werden. Ein günstiger therapeutischer Effekt ist ferner bei verschiedenen funktionellen Beschwerden zu erwarten, die zu Oberbauchschmerzen, Übelkeit, Völlegefühl und sonstigen Mißempfindungen führen. Hierzu gehören die Symptome bei Ulcus ventriculi und Ulcus duodeni, bei Gastritis und nervörsem Reizmagen. Ebenso wird die in der Röntgendiagnostik des Magen-Darm-Kanales erwünschte gesteigerte Magenpassage der Kontrastmittel erreicht.

Die Arzneimittelzubereitungen enthalten die Substanzen der Formel I oder deren pharmakologisch verträgliche Salze als Wirkstoff in Kombination mit üblichen pharmakologisch verträglichen Trägerstoffen und/oder Verdünnungsmitteln. Die Arzneimittel können oral oder parenteral verabreicht werden und liegen als Tabletten, Kapseln, Sirups, Trockenpulver, injizierbare und infundierbare Lösungen oder Suspensionen vor. Sie können aber auch als Suppositorien konfektioniert werden. Im allgemeinen werden oral verabreichbare Präparate bevorzugt.

Die Dosierung der erfindungsgemäßen Arzneipräparate hängt von verschiedenen Faktoren ab, wie von der Art und der Schwere der Krankheit oder von der verwendeten Verbindung. Im allgemeinen genügt bei oraler Gabe eine Einzeldosis von 1 bis 50, insbesondere 2 bis 20 mg, um zufriedenstellende Ergebnisse zu erhalten.

## Beispiel I

Kapseln mit 10 mg 1-(β-Dimethylaminoäthyl)-2-methoxycarbonyl-3-methoxy-indol-hydrochlorid als Wirkstoff Zusammensetzung:

| | |
|---|---|
| Wirkstoff | 10 Teile |
| Lactose | 65 Teile |
| Maisstärke, getrocknet | 40 Teile |
| lösliche Stärke | 4 Teile |
| Magnesiumstearat | 1 Teil |
| | 120 Teile |

### Herstellungsvorschrift:

Der Wirkstoff wird mit Lactose und Maisstärke vermischt. Die entstandene Mischung wird mit einer 15%igen wäßrigen Lösung der löslichen Stärke durchfeuchtet und granuliert. Die feuchte Masse wird durch ein 1,6 mm-Sieb passiert, bei 40°C auf Horden getrocknet und anschließend durch ein 1,0 mm-Sieb passiert. Nach dem Vermischen des Granulats mit Magnesiumstearat wird die entstandene Mischung in Mengen von 120 mg verkapselt, so daß jede Kapsel 10 mg Wirkstoff enthält.

Die nachfolgenden Beispiele dienen zur Erläuterung der Erfindung.

## Beispiel 1

20,5 g 2-Methoxycarbonyl-3-methoxy-indol werden in 100 ml Dimethylformamid gelöst und bei 0°C unter Rühren portionsweise mit 3 g Natriumhydrid (80%ig) versetzt. Nach Erwärmen auf Raumtemperatur fügt man 20 g 1-Brom-3-chlorpropan hinzu, erhitzt weitere 12 Std. auf 80°C und zieht das Lösungsmittel ab. Man arbeitet mit Äthylacetat/Wasser wie üblich auf. Das erhaltene Rohprodukt wird einer Kugelrohrdestillation unterworfen. Man erhält 24,7 g des ölig anfallenden 1-(γ-Chlorpropyl)-2-methoxycarbonyl-3-methoxy-indol. Dieses wird in 100 ml Dimethylformamid gelöst und mit 20 g Diäthylamin 5 Std. auf 60°C erhitzt. Nach Abziehen des Lösungsmittels und Versetzen des Rückstands mit 10%iger Salzsäure wird mit Äthylacetat extrahiert. Anschließend wird die wäßrige Phase mit Sodalösung alkalisch gemacht, mit Äthylacetat extrahiert, die organische Phase über Natriumsulfat getrocknet, filtriert und unter vermindertem Druck zur Trockne eingedampft. Als Rückstand erhält man 25,8 g (81% d. Th.) 1-(γ-Diäthylaminopropyl)-2-methoxycarbonyl-3-methoxy-indol als Öl. Die Substanz fällt als Maleinat in kristalliner Form mit Fp. 110 – 112°C an.

Unter entsprechenden Reaktionsbedingungen kann man das 1-(γ-Chlorpropyl)-2-methoxycarbonyl-3-methoxy-indol mit Methylamin oder Ammoniak umsetzen und gegebenenfalls durch darauffolgende Umsetzung mit einer Säure folgende Verbindungen erhalten:

| | Fp. °C |
|---|---|
| 1-(γ-Methylaminopropyl)-2-methoxycarbonyl-3-methoxy-indol, IR: 1695, 3300 $cm^{-1}$ (Carbonyl/NH) | Öl |
| 1-(γ-Aminopropyl)-2-methoxycarbonyl-3-methoxy-indol-hydrochlorid | 184–186 |

Beispiel 2

Entsprechend dem obigen Beispiel erhält man aus 20,5 g 2-Methoxycarbonyl-3-methoxy-indol, gelöst in 100 ml Dimethylformamid, mit 3 g Natriumhydrid (80%ig) und 22 g 1-Brom-4-chlorbutan 25,1 g 1-(δ-Chlorbutyl)-2-methoxycarbonyl-3-methoxy-indol als Öl. Dieses wird in 100 ml Dimethylformamid gelöst, bei 50° C mit Dimethylamin umgesetzt und als Toluolsulfonat in kristalliner Form gewonnen. Man erhält 29,4 g (62% d. Th.) 1-(δ-Dimethylaminobutyl)-2-methoxycarbonyl-3-methoxyindol-toluol-4-sulfonat mit Fp. 133 – 135° C.

Beispiel 3

20,5 g 2-Methoxycarbonyl-3-methoxy-indol werden in 60 ml Dimethylformamid gelöst. Unter Rühren werden bei Eiskühlung portionsweise 3 g Natriumhydrid (80%ig) hinzugegeben. Nach 15 min werden 12 g 1-Dimethylamino-2-chloräthan hinzugefügt, danach wird 2 h auf 60° C erhitzt und anschließend unter vermindertem Druck eingedampft. Der Rückstand wird mit 10%iger Salzsäure angesäuert und mit Äthylacetat extrahiert. Anschließend wird die wäßrige Phase mit Sodalösung alkalisch gemacht, mit Äthylacetat extrahiert, die organische Phase über Natriumsulfat getrocknet, filtriert und unter vermindertem Druck zur Trockne eingedampft. Als Rückstand erhält man 23,7 g 1-(β-Dimethylaminoäthyl)-2-methoxycarbonyl-3-methoxy-indol in öliger Form (86% d. Th.).
Zur Salzbildung wird das Öl in 100 ml Methanol gelöst und Chlorwasserstoff gasförmig in die Lösung geleitet. Das ausgefallene Salz wird abgesaugt, mit Methanol und Äther nachgewaschen und getrocknet. Man erhält die vorgenannte Verbindung als Monohydrochlorid mit Fp. 202 – 203° C (Zers.).

Beispiel 4

Entsprechend den Beispielen 1 bis 3 kann man, ausgehend von den Alkalisalzen der entsprechenden Indolderivate, folgende Verbindungen herstellen:

| | Fp. °C |
|---|---|
| 1-(β-Diäthylaminoäthyl)-2-äthoxycarbonyl-3-äthoxy-indol-maleinat | 110—112 |
| 1-(β-Dimethylaminoäthyl)-2-isopropyloxycarbonyl-3-methoxy-indol, IR: 1685 $cm^{-1}$ (Carbonyl) | Öl |
| 1-(β-Dimethylaminoäthyl)-2-methoxycarbonyl-3-isopropyloxy-5-methyl-indol-hydrochlorid | 206—208 |
| 1-(β-Dimethylaminoäthyl)-2-methoxycarbonyl-3-methoxy-6-methyl-indol-hydrochlorid | 198—200 |
| 1-(β-Dimethylaminoäthyl)-2-methoxycarbonyl-3-äthoxy-5-chlor-indol-hydrochlorid | 175—177 |
| 1-(β-Dimethylaminoäthyl)-2-methoxycarbonyl-3-benzyloxy-5-chlor-indol-toluol-4-sulfonat | 175—177 |
| 1-(β-Dimethylaminoäthyl)-2-äthoxycarbonyl-3-äthoxy-6-chlor-indol-toluol-4-sulfonat | 152—154 |
| 1-(β-Dimethylaminoäthyl)-2-methoxycarbonyl-3-methoxy-5-brom-indol-hydrochlorid | 190—191 |
| 1-(β-Dimethylaminoäthyl)-2-methoxycarbonyl-3-methoxy-5-nitro-indol, IR: 1690 $cm^{-1}$ (C=O) | Öl |
| 1-(β-Dimethylaminoäthyl)-2-methoxycarbonyl-3,5-dimethoxy-indol-hydrochlorid | 182—184 |
| 1-(β-Dimethylaminoäthyl)-2-methoxycarbonyl-3,5,6-trimethoxy-indol-hydrochlorid | 203—204 |
| 1-(β-Dimethylaminoäthyl)-2-methoxycarbonyl-3-methoxy-5-brom-6-methyl-indol-hydrochlorid | 200—202 |
| 1-(β-Dimethylaminoäthyl)-2-cyano-3-methoxy-indol, IR: 2230 $cm^{-1}$ (Nitril) | Öl |
| 1-(γ-Dimethylaminopropyl)-2-methoxycarbonyl-3-methoxy-indol-hydrochlorid | 154—155 |

| | Fp. °C |
|---|---|
| 1-(β-Dimethylaminopropyl)-2-methoxycarbonyl-3-methoxy-indol-hydrochlorid | 186–188 |
| 1-(γ-Diisopropylaminopropyl)-2-methoxy-carbonyl-3-methoxy-indol, IR: 1700 $cm^{-1}$ (Carbonyl) | Öl |
| 1-(γ-Pyrrolidinopropyl)-2-methoxycarbonyl-3-methoxy-indol, IR: 1700 $cm^{-1}$ (Carbonyl) | Öl |
| 1-(γ-Piperidinopropyl)-2-methoxycarbonyl-3-methoxy-indol-maleinat | 104–106 |
| 1-(γ-Isopropylaminopropyl)-2-methoxycarbonyl-3-methoxy-indol-hydrochlorid | 148–149 |

Beispiel 5

23,3 g 2-Methoxycarbonyl-3-acetoxy-indol, gelöst in Dimethylformamid, werden entsprechend Beispiel 2 mit 3 g Natriumhydrid und nachfolgend mit 1-Dimethylamino-2-chloräthan zum 1-(β-Dimethylaminoäthyl)-2-methoxycarbonyl-3-acetoxy-indol, Fp. 165–167° C, umgesetzt. Das Produkt wird in 150 ml Methanol gelöst, mit einer Lösung von 10 g Natriumcarbonat in 100 ml Wasser versetzt und eine Stunde bei Raumtemperatur gerührt. Der pH-Wert wird danach mit verdünnter Salzsäure auf 8 eingestellt, das Lösungsmittel weitgehend abgezogen und der Rückstand wie üblich mit Äthylacetat/Wasser aufgearbeitet. Das 1-(β-Dimethylaminoäthyl)-2-methoxycarbonyl-3-hydroxy-indol kristallisiert als Maleinat aus Äthylacetat/Äther, Fp. 156–158° C. Die Ausbeute beträgt 26,8 g (71% d. Th.).

Beispiel 6

2 g 1-(β-Dimethylaminoäthyl)-2-methoxycarbonyl-3-benzyloxy-5-chlor-indol werden in 50 ml Äthylacetat gelöst und mit 2 g Palladium/Kohle (5% Pd) bei Raumtemperatur und Atmosphärendruck hydriert. Nach 2 Stunden filtriert man vom Katalysator ab und engt die Reaktionslösung ein. Es verbleiben 1,5 g 1-(β-Dimethylaminoäthyl)-2-methoxycarbonyl-3-hydroxy-5-chlor-indol als leicht gelb gefärbtes Öl.

IR: 1710 $cm^{-1}$ Carbonyl
3300 $cm^{-1}$ Hydroxy

Beispiel 7

3,5 g 1-γ-Diäthylaminopropyl)-2-äthoxycarbonyl-3-äthoxy-indol werden in 50 ml Äthanol gelöst und mit 2,5 ml konzentrierter Salzsäure 12 Stunden unter Rückfluß gekocht. Danach wird das Lösungsmittel weitgehend abgezogen, der Rückstand mit Natriumcarbonatlösung bei Raumtemperatur alakalisch gestellt und mit Dichlormethan wie üblich aufgearbeitet. Das 1-(γ-Diäthylaminopropyl)-2-äthoxycarbonyl-3-hydroxy-indol fällt ölig mit Ausbeute von 2,7 g (85% d. Th.) an.

IR: 1715 $cm^{-1}$ Carbonyl
3280 $cm^{-1}$ Hydroxy

Beispiel 8

27,6 g 1-(β-Dimethylaminoäthyl)-2-methoxycarbonyl-3-methoxy-indol werden in 150 ml Methanol gelöst und mit einer Lösung von 4,4 g Natriumhydroxid in 40 ml Wasser versetzt. Es wird eine Stunde unter Rückfluß erhitzt, mit Essigsäure auf pH 7 eingestellt und unter vermindertem Druck eingeengt. Der Rückstand wird mit Wasser aufgenommen, mit Ammoniumsulfat gesättigt und mit Äthylacetat extrahiert. Die organische Phase wird getrocknet, filtriert und eingedampft, der Rückstand kristallisiert aus Methanol. Es werden 24,7 g (94% d. Th.) 1-(β-Dimethylaminoäthyl)-2-hydroxycarbonyl-3-methoxy-indol mit Fp. 188–190° C erhalten. Ebenso erhält man:

1-(γ-Dimethylaminopropyl)-2-hydroxycarbonyl-3-methoxy-indol, Öl.
1-(β-Dimethylaminoäthyl)-2-hydroxycarbonyl-3-äthoxy-indol, Fp. 160–165° C.
1-(β-Dimethylaminoäthyl)-2-hydroxycarbonyl-3-benzyloxy-indol, Öl.
1-(β-Dimethylaminoäthyl)-2-hydroxycarbonyl-3-methoxy-5-chlor-indol, Öl.
1-(β-Dimethylaminoäthyl)-2-hydroxycarbonyl-3-methoxy-6-chlor-indol, Öl.
1-(β-Dimethylaminoäthyl)-2-hydroxycarbonyl-3-methoxy-5-methyl-indol, Fp. 159–163° C.
1-(β-Dimethylaminoäthyl)-2-hydroxycarbonyl-3-methoxy-5-methoxy-indol, Öl.

1-(β-Dimethylaminoäthyl)-2-hydroxycarbonyl-3-methoxy-5,6-dimethoxy-indol, Öl.
1-(β-Diäthylaminoäthyl)-2-hydroxycarbonyl-3-methoxy-indol, Öl.
1-(β-Methylaminoäthyl)-2-hydroxycarbonyl-3-methoxy-indol, Öl.

Beispiel 9

2,6 g 1-(β-Dimethylaminoäthyl)-2-hydroxycarbonyl-3-methoxy-indol werden in 50 ml Dichlormethan und 1 g Triäthylamin gelöst. Hierzu gibt man 10 ml konzentrierten wäßrigen Ammoniak, kühlt auf 0° C herab und gibt unter Rühren 1,1 g Chlorameisensäureäthylester hinzu. Man läßt die Reaktionsmischung wieder auf Raumtemperatur erwärmen und arbeitet mit Dichlormethan/Wasser wie üblich auf. Das 1-(β-Dimethylaminoäthyl)-2-aminocarbonyl-3-methoxy-indol kristallisiert aus Methylenchlorid/Äther mit Fp. 113 – 115° C. Die Ausbeute beträgt 1,7 g (65% d. Th.).

Entsprechend erhält man bei der Umsetzung von 1-(β-Dimethylaminoäthyl)-2-hydroxycarbonyl-3-methoxy-indol mit Methylamin das 1-(β-Dimethylaminoäthyl)-2-methylaminocarbonyl-3-methoxy-indol, Fp. 175° C (zers.).

Beispiel 10

2,6 g 1-(β-Dimethylaminoäthyl)-2-hydroxycarbonyl-3-methoxy-indol und 2,3 g Dicyclohexylcarbodiimid werden in 50 ml Dichlormethan gelöst und auf 0° C abgekühlt. Nach 30 min gibt man unter Rühren 5 ml Diäthylamin hinzu, läßt auf Raumtemperatur erwärmen, engt ein und arbeitet mit Äthylacetat/Wasser wie üblich auf. Der Rückstand wird an Aluminiumoxid mit Cyclohexan/Äthylacetat chromatographiert. Es resultieren 1,7 g (53% d. Th.) 1-(β-Dimethylaminoäthyl)-2-diäthylaminocarbonyl-3-methoxy-indol als farbloses Öl. IR: 1620 $cm^{-1}$ (Carbonyl).

Beispiel 11

2,9 g 1-(β-Dimethylaminoäthyl)-2-methoxycarbonyl-3-methoxy-5-methyl-indol werden in 65 ml Diisopropylamin 5 Std. unter Rückfluß erhitzt. Man zieht das Lösungsmittel ab und reinigt das verbleibende Öl säulenchromatographisch (Kieselgel, Elutionsmittel: Äther/Petroläther).

Das gewonnene 1-(β-Dimethylaminoäthyl)-2-diisopropylaminocarbonyl-3-methoxy-5-methyl-indol fällt als farbloses Öl mit einer Ausbeute von 1,7 g (47% d. Th.) an. IR: 1615 $cm^{-1}$ (Carbonyl).

Auf die in den Beispielen 9 – 11 beschriebene Weise können beispielsweise auch folgende Verbindungen hergestellt werden:

1-(β-Dimethylaminoäthyl)-2-diäthylaminocarbonyl-3-methoxy-6-methyl-indol
1-(β-Dimethylaminoäthyl)-2-diäthylaminocarbonyl-3-äthoxy-5-chlor-indol
1-(β-Dimethylaminoäthyl)-2-diäthylaminocarbonyl-3-isopropyloxy-5-methyl-indol
1-(β-Dimethylaminoäthyl)-2-diäthylaminocarbonyl-3-benzyloxy-5-chlor-indol
1-(γ-Isopropylaminopropyl)-2-diäthylaminocarbonyl-3-methoxy-indol
1-(δ-Dimethylaminobutyl)-2-diäthylaminocarbonyl-3-methoxy-indol
1-(γ-N-Piperidinopropyl)-2-diäthylaminocarbonyl-3-methoxy-indol

**Patentansprüche**

1. N-Aminoalkylindol-Derivate der Formel I

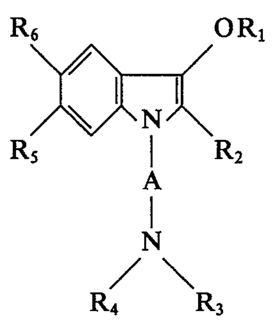

,

worin $R_1$ ein Wasserstoffatom, eine gegebenenfalls mit einem Phenylrest substituierte Alkylgruppe mit 1 bis 4 Kohlenstoffatomen oder ein Acetylrest und $R_2$ die Hydroxycarbonylgruppe, eine

Alkoxycarbonylgruppe mit 1 bis 4 Kohlenstoffatomen, die Cyanogruppe oder eine Amino-, Monoalkylamino- oder Dialkylaminocarbonylgruppe mit Alkylresten mit 1 bis 4 Kohlenstoffatomen bedeuten, wobei, wenn $R_2$ die Hydroxycarbonylgruppe ist, $R_1$ kein Wasserstoffatom bedeutet, A eine $C_2-C_5$-Alkylengruppe ist, $R_3$ und $R_4$ gleich oder verschieden sind und Wasserstoffatome oder Alkylreste mit 1 bis 4 Kohlenstoffatomen bedeuten, oder $R_3$ und $R_4$ gemeinsam mit dem Stickstoffatom, an das sie gebunden sind, eine cyclische Gruppe mit 5 bis 7 Kettengliedern bilden, $R_5$ und $R_6$ gleich oder verschieden sind und Wasserstoffatome, Halogenatome oder Alkyl- oder Alkoxygruppen mit je 1 bis 3 Kohlenstoffatomen bedeuten oder einer der beiden Reste $R_5$ und $R_6$ eine Trifluormethyl- oder Nitrogruppe und der andere ein Wasserstoffatom bedeutet, sowie deren Säureadditionssalze.

2. 1-($\beta$-Dimethylaminoäthyl)-2-methoxycarbonyl-3-methoxyindole der allgemeinen Formel I, in welcher $R_6$ Wasserstoff, Brom oder Methoxy und $R_5$ Wasserstoff, Methyl oder Methoxy sind oder $R_6$ Nitro und $R_5$ Wasserstoff sind.

3. 1-($\beta$-Dimethylaminoäthyl)-2-methoxycarbonylindole der allgemeinen Formel I, in welcher $OR_1$ Äthoxy, Isopropyloxy oder Benzyloxy und $R_6$ Chlor oder Methyl sind.

4. 1-($\beta$-Dimethylaminoäthyl)-2-äthoxycarbonyl-3-äthoxy-6-chlor-indol.

5. 1-($\beta$-Dimethylaminoäthyl)-2-isopropyloxycarbonyl-3-methoxy-indol.

6. 1-($\beta$-Diäthylaminoäthyl)-2-äthoxycarbonyl-3-äthoxy-indol.

7. 2-Methoxycarbonyl-3-methoxy-indole der allgemeinen Formel I, in welcher die

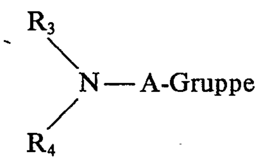

$\beta$-Dimethylaminopropyl, $\gamma$-Aminopropyl, $\gamma$-Methylaminopropyl, $\gamma$-Dimethylaminopropyl, $\gamma$-Diäthylaminopropyl, $\gamma$-Isopropylaminopropyl, $\gamma$-Diisopropylaminopropyl, $\gamma$-N-Pyrrolidinopropyl, $\gamma$-N-Piperidinopropyl oder $\gamma$-Dimethylaminobutyl ist.

8. 1-($\beta$-Dimethylaminoäthyl)-2-hydroxycarbonyl-3-methoxy-indol.

9. 1-($\beta$-Dimethylaminoäthyl)-3-methoxy-indol der allgemeinen Formel I, in welcher $R_2$ Aminocarbonyl, Methylaminocarbonyl, Diäthylaminocarbonyl oder Diisopropylaminocarbonyl und $R_6$ Wasserstoff oder Methyl sind.

10. 1-($\beta$-Dimethylaminoäthyl)-2-methoxycarbonyl-3-hydroxy-indole der allgemeinen Formel I, in welcher $R_6$ Wasserstoff oder Chlor ist.

11. 1-($\gamma$-Diäthylaminopropyl)-2-äthoxycarbonyl-3-hydroxy-indol.

12. Verfahren zur Herstellung von N-Aminoalkylindol-Derivaten der Formel I

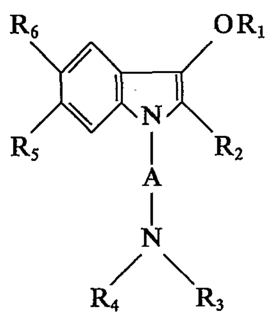

worin $R_1$ ein Wasserstoffatom, eine gegebenenfalls mit einem Phenylrest substituierte Alkylgruppe mit 1 bis 4 Kohlenstoffatomen oder ein Acetylrest und $R_2$ die Hydroxycarbonylgruppe, eine Alkoxycarbonylgruppe mit 1 bis 4 Kohlenstoffatomen, die Cyanogruppe oder eine Amino-, Monoalkylamino- oder Dialkylaminocarbonylgruppe mit Alkylresten mit 1 bis 4 Kohlenstoffatomen bedeuten, wobei, wenn $R_2$ die Hydroxycarbonylgruppe ist, $R_1$ kein Wasserstoffatom bedeutet, A eine $C_1-C_5$-Alkylengruppe ist, $R_3$ und $R_4$ gleich oder verschieden sind und Wasserstoffatome oder Alkylreste mit 1 bis 4 Kohlenstoffatomen bedeuten, oder $R_3$ und $R_4$ gemeinsam mit dem Stickstoffatom, an das sie gebunden sind, eine cyclische Gruppe mit 5 bis 7 Kettengliedern bilden, $R_5$ und $R_6$ gleich oder verschieden sind und Wasserstoffatome, Halogenatome oder Alkyl- oder Alkoxygruppen mit je 1 bis 3 Kohlenstoffatomen bedeuten oder einer der beiden Reste $R_5$ und $R_6$ eine Trifluormethyl- oder Nitrogruppe und der andere ein Wasserstoffatom bedeutet, sowie deren Säureadditionssalze, dadurch gekennzeichnet, daß man ein Alkalimetallsalz des Indols der Formel II

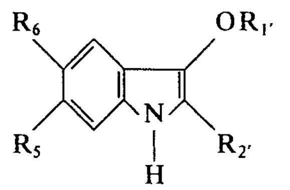

worin $R_5$ und $R_6$ die obige Bedeutung haben, $R_{1'}$ eine gegebenenfalls mit einem Phenylrest substituierte Alkylgruppe oder eine Acetylgruppe und $R_{2'}$ eine Alkoxycarbonylgruppe der obigen Bedeutung oder die Cyanogruppe sind, in einem inerten Lösungsmittel

a) nacheinander mit einem Dihalogenalkan der Formel III

$$X_1 - A - X_2$$

worin A die obengenannte Bedeutung hat, $X_1$ und $X_2$ gleich oder verschieden sein können und Halogen bedeuten, und einem Amin der Formel IV

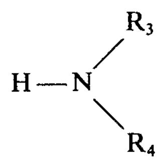

worin $R_3$ und $R_4$ die obengenannte Bedeutung haben, oder allein mit einem Halogenalkylamin der Formel V

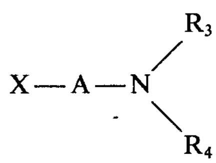

worin A die obengenannte Bedeutung besitzt, $R_3$ und $R_4$ die obengenannten Bedeutungen außer Wasserstoffatom haben und X ein Halogenatom ist, umsetzt zu Verbindungen der Formel I

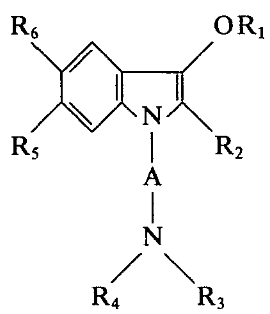

worin A, $R_3$, $R_4$, $R_5$ und $R_6$ die obigen Bedeutungen haben, und $R_1$ eine gegebenenfalls mit einem Phenylrest substituierte Alkylgruppe oder eine Acetylgruppe und $R_2$ eine Cyano- oder Alkoxycarbonylgruppe sind,

b) gewünschtenfalls eine Cyano- oder Alkoxycarbonylgruppe $R_2$ in die Hydroxycarbonylgruppe $R_2$ überführt,

c) gewünschtenfalls eine Cyano, Alkoxycarbonyl- oder Hydroxycarbonylgruppe $R_2$ in eine gegebenenfalls mono- oder disubstituierte Aminocarbonylgruppe $R_2$ überführt,

d) gewünschtenfalls in den nach a) oder c) erhaltenen Verbindungen, worin $R_1$ Acetyl bedeutet, die Acetylgruppe $R_1$ hydrolytisch abspaltet und die 3-Hydroxyverbindungen isoliert,

e) gewünschtenfalls in den nach a) oder c) erhaltenen Verbindungen, worin $R_1$ Benzyl bedeutet, die Benzyloxygruppe hydrogenolytisch spaltet und die 3-Hydroxyverbindungen isoliert,

f) gewünschtenfalls in den nach a) oder c) erhaltenen Verbindungen, worin $R_1$ Alkyl bedeutet, die Alkoxygruppe hydrolytisch spaltet und die 3-Hydroxyverbindungen isoliert und

g) die so erhaltenen Basen isoliert und diese gewünschtenfalls in die entsprechenden Säureadditionssalze überführt.

13. Arzneimittel, bestehend aus einer oder mehreren Verbindungen gemäß Anspruch 1 und üblichen pharmazeutischen Hilfs- und/oder Trägerstoffen.

## Claims

1. N-aminoalkylindole derivatives of the formula I

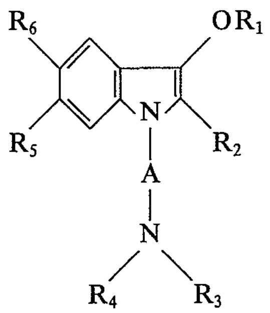

wherein $R_1$ is a hydrogen atom, an alkyl radical with 1 to 4 carbon atoms, optionally substituted with a phenyl radical, or an acetyl radical, and $R_2$ is the hydroxycarbonyl radical, an alkoxycarbonyl radical with 1 to 4 carbon atoms, the cyano group or an amino-, monoalkylamino- or dialkylaminocarbonyl radical, with alkyl radicals of 1 to 4 carbon atoms, whereby $R_1$ is not a hydrogen atom when $R_2$ is a hydroxycarbonyl radical, A is a $C_2-C_5$-alkylene radical, $R_3$ and $R_4$ are the same or different and are hydrogen atoms or alkyl radicals with 1 to 4 carbon atoms, or $R_3$ and $R_4$ together eith the nitrogen atom to which they are bonded, form a cyclic ring with 5 to 7 chain-members, $R_5$ and $R_6$ are the same or different and are hydrogen atoms, halogen atoms or alkyl or alkoxy radicals each with 1 to 3 carbon atoms, or one of the two substituents $R_5$ and $R_6$ is a trifluoromethyl radical or nitro group and the other is a hydrogen atom, as well as their acid addition salts.

2. 1-(β-Dimethylaminoethyl)-2-methoxycarbonyl-3-methoxy-indoles of the general formula I, wherein $R_6$ is hydrogen, bromine or methoxy and $R_5$ is hydrogen, methyl or methoxy, or $R_6$ is nitro and $R_5$ is hydrogen.

3. 1-(β-Dimethylaminoethyl)-2-methoxycarbonyl-indoles of the general formula I, wherein $OR_1$ is ethoxy, isopropyloxy or benzyloxy, and $R_6$ is chlorine or methyl.

4. 1-(β-Dimethylaminoethyl)-2-ethoxycarbonyl-3-ethoxy-6-chloro-indole.

5. 1-(β-Dimethylaminoethyl)-2-isopropyloxycarbonyl-3-methoxy-indole.

6. 1-(β-Diethylaminoethyl)-2-ethoxycarbonyl-3-ethoxy-indole.

7. 2-Methoxycarbonyl-3-methoxy-indoles of the general formula I, wherein the

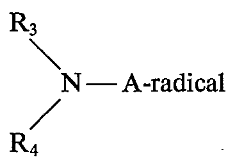

is β-dimethylaminopropyl, γ-aminopropyl, γ-methylaminopropyl, γ-dimethylaminopropyl, γ-diethylaminopropyl, γ-isopropylaminopropyl, γ-diisopropylaminopropyl, γ-N-pyrrolidinopropyl, γ-N-piperidinopropyl or γ-dimethylaminobutyl.

8. 1-(β-Dimethylaminoethyl)-2-hydroxycarbonyl-3-methoxy-indole.

9. 1-(β-Dimethylaminoethyl)-3-methoxy-indoles of the general formula I, wherein $R_2$ is aminocarbonyl, methylaminocarbonyl, diethylaminocarbonyl or diisopropylaminocarbonyl, and $R_6$ is hydrogen or methyl.

10. 1-(β-Dimethylaminoethyl)-2-methoxycarbonyl-3-hydroxy-indoles of the general formula I, wherein $R_6$ is hydrogen or chlorine.

11. 1-(γ-Diethylaminopropyl)-2-ethoxycarbonyl-3-hydroxy-indole.

12. A process for the production of N-aminoalkylindole derivatives of the formula I

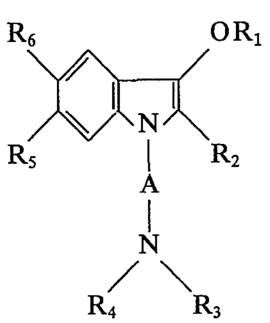

wherein $R_1$ is a hydrogen atom, an alkyl radical with 1 to 4 carbon atoms, optionally substituted with a phenyl radical, or an acetyl radical, and $R_2$ is the hydroxycarbonyl radical, an alkoxycarbonyl radical with 1 to 4 carbon atoms, the cyano group or an amino-, monoalkylamino- or dialkylaminocarbonyl radical, with alkyl radicals of 1 to 4 carbon atoms, whereby $R_1$ is not a hydrogen atom when $R_2$ is a hydroxycarbonyl radical, A is a $C_2-C_5$-alkylene radical, $R_3$ and $R_4$ are the same or different and are hydrogenatoms or alkyl radicals with 1 to 4 carbon atoms, or $R_3$ and $R_4$ together with the nitrogen atom to which they are bonded form a cyclic ring eith 5 to 7 chain members, $R_5$ and $R_6$ are the same or different and are hydrogen atoms, halogen atoms or alkyl or alkoxy radicals each with 1 to 3 carbon atoms, or one of the two substituents $R_5$ and $R_6$ is a trifluoromethyl radical or nitro group and the other is a hydrogen atom: as well as their acid addition salts, characterised in that an alkali metal salt of an indole of the formula II

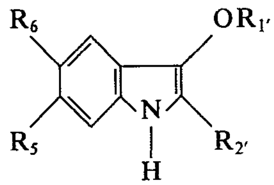

where $R_5$ and $R_6$ have the above meanings, $R_1$, is an alkyl radical with 1 to 4 carbon atoms, optionally substituted with a phenyl radical, or an acetyl group and $R_2$, is an alkoxycarbonyl radical having the above meaning or a cyano group, is reacted in an inert solvent

(a) successively with a dihaloalkane of the formula III

$$X_1-A-X_2$$

wherein A has the above meaning, $X_1$ and $X_2$ can be the same or different and are halogen and with an amine of the formula IV

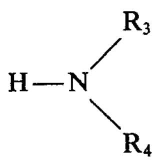

where $R_3$ and $R_4$ have the above meanings or alone with a haloalkylamine of the formula V

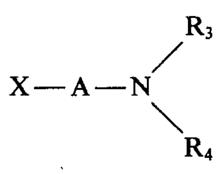

where A has the above meanings, $R_3$ and $R_4$ have the above meanings, with the exception of the hydrogen atom, and X is a halogen atom, to give a compound of the formula I

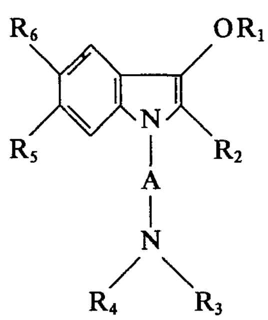

where A, $R_3$, $R_4$, $R_5$ and $R_6$ have the above meanings, and $R_1$ is an alkyl radical optionally substituted with a phenyl radical or an acetyl radical and $R_2$ is a cyano group or an alkoxycarbonyl radical,

(b) if desired a cyano group or alkoxycarbonyl radical $R_2$ is converted into the hydroxycarbonyl radical, or

(c) if desired a cyano, alkoxycarbonyl- or hydroxycarbonyl radical $R_2$ is converted into a optionally mono- or disubstituted aminocarbonyl radical,

(d) if desired in the compounds abtained according to (a) or (c) where $R_1$ is an acetyl radical, the

acetyl radical is splitt off hydrolytically and the 3-hydroxy compounds isolated,

(e) if desired in the compounds obtained according to (a) or (c) where $R_1$ is benzyl, the benzyloxy radical is splitt off hydrogenolytically and the 3-hydroxy compounds isolated,

(f) if desired in the compounds obtained according to (a) or (c) where $R_1$ is alkyl the alkoxy radical is splitt offf hydrolytically and the 3-hydroxycompounds isloated, and

(g) the bases obtained are isolated and, if desired, converted into the corresponding acid addition salts.

13. A pharmaceutical composition, comprising one or more of compounds according to Claim 1 and conventional pharmaceutical auxilliary and/or carrier materials.

**Revendications**

1. Dérivés de N-aminoalkylindole de formule I:

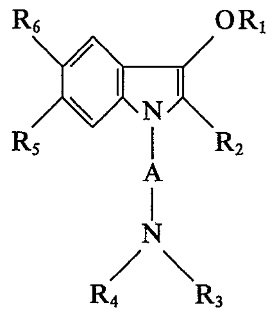

dans laquelle $R_1$ représente un atome d'hydrogène, un groupe alkyle contenant 1 à 4 atomes de carbone et éventuellement substitué par un groupe phényle, ou encore un groupe acétyle et $R_2$ représente le groupe hydroxycarbonyle, un groupe alcoxycarbonyle contenant 1 à 4 atomes de carbone, le groupe cyano ou un groupe amino-, monoalkylamino- ou dialkylaminocarbonyle dont les groupes alkyle contiennent 1 à 4 atomes de carbone et, si $R_2$ représente le groupe hydroxycarbonyle, $R_1$ ne représente pas un atome d'hydrogène, A représente un groupe alkylène en $C_2-C_5$, $R_3$ et $R_4$ sont identiques ou différents et représentent des atomes d'hydrogène ou des groupes alkyle contenant 1 à 4 atomes de carbone, ou encore $R_3$ et $R_4$ ensemble avec l'atome d'azote auquel ils sont reliés, forment un groupe cyclique contenant 5 à 7 chaînons, $R_5$ et $R_6$ sont identiques ou différents et représentent des atomes d'hydrogène, des atomes d'halogènes ou des groupes alkyle ou alcoxy contenant chacun 1 à 3 atomes de carbone, ou un des deux radicaux $R_5$ et $R_6$ représente un groupe trifluorométhyle ou nitro, tandis que l'autre représente un atome d'hydrogène, ainsi que les sels d'addition d'acide de ces dérivés.

2. 1-($\beta$-diméthylaminoéthyl)-2-méthoxycarbonyl-3-méthoxy-indoles de formule générale I dans laquelle $R_6$ représente un atome d'hydrogène, un atome de brome ou un groupe méthoxy et $R_5$ représente un atome d'hydrogène, un groupe méthyle ou un groupe méthoxy ou encore $R_6$ représente un groupe nitro et $R_5$ représente un atome d'hydrogène.

3. 1-($\beta$-diméthylaminoéthyl)-2-méthoxycarbonyl-indoles de formule générale I dans laquelle $OR_1$ représente un groupe éthoxy, un groupe isopropyloxy ou un groupe benzyloxy et $R_6$ représente un atome de chlore ou un groupe méthyle.

4. 1-($\beta$-diméthylaminoéthyl)-2-éthoxycarbonyl-3-éthoxy-6-chloro-indole.

5. 1-($\beta$-diméthylaminoéthyl)-2-isopropyloxycarbonyl-3-méthoxy-indole.

6. 1-($\beta$-diéthylaminoéthyl)-2-éthoxycarbonyl-3-éthoxy-indole.

7. 2-méthoxycarbonyl-3-méthoxy-indoles des formule générale I dans laquelle le groupe

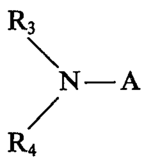

est le groupe $\beta$-diméthylaminopropyle, le groupe $\gamma$-aminopropyle, le groupe $\gamma$-méthylaminopropyle, le groupe $\gamma$-diméthylaminopropyle, le groupe $\gamma$-diéthylaminopropyle, le groupe $\gamma$-isopropylaminopropyle, le groupe $\gamma$-diisopropylaminopropyle, le groupe $\gamma$-N-pyrrolidinopropyle, le groupe $\gamma$-N-pipéridinopropyle ou le groupe $\gamma$-diméthylaminobutyle.

8. 1-($\beta$-diméthylaminoéthyl)-2-hydroxycarbonyl-3-méthoxy-indole.

9. 1-($\beta$-diméthylaminoéthyl)-3-méthoxy-indole de formule générale I dans laquelle $R_2$ représente un groupe aminocarbonyle, un groupe méthylaminocarbonyle, un groupe diéthylaminocarbonyle ou un groupe diisopropylaminocarbonyle et $R_6$ représente un atome d'hydrogène ou un groupe méthyle.

10. 1-(β-diméthylaminoéthyl)-2-méthoxycarbonyl-3-hydroxy-indoles de formule générale I dans laquelle $R_6$ représente un atome d'hydrogène ou un atome de chlore.

11. 1-(γ-diéthylaminopropyl)-2-éthoxycarbonyl-3-hydroxy-indole.

12. Procédé de préparation de dérivés de N-aminoalkylindole de formule I:

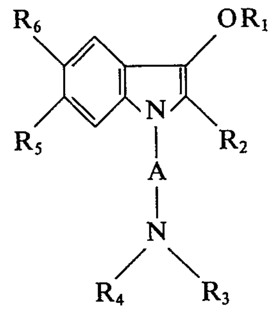

dans laquelle $R_1$ représente un atome d'hydrogène, un groupe alkyle contenant 1 à 4 atomes de carbone et éventuellement substitué par un groupe phényle, ou encore un groupe acétyle et $R_2$ représente le groupe hydroxycarbonyle, un groupe alcoxycarbonyle contenant 1 à 4 atomes de carbone, le groupe cyano ou un groupe amino-, monoalkylamino- ou dialkylaminocarbonyle dont les groupes alkyle contiennent 1 à 4 atomes de carbone et, si $R_2$ représente le groupe hydroxycarbonyle, $R_1$ ne représente pas un atome d'hydrogène, A représente un groupe alkylène en $C_2-C_5$, $R_3$ et $R_4$ sont identiques ou différents et représentent des atomes d'hydrogène ou des groupes alkyle contenant 1 à 4 atomes de carbone, ou encore $R_3$ et $R_4$ ensemble avec l'atome d'azote auquel ils sont reliés, forment un groupe cyclique contenant 5 à 7 chaînons, $R_5$ et $R_6$ sont identiques ou différents et représentent des atomes d'hydrogène, des atomes d'halogènes ou des groupes alkyle ou alcoxy contenant chacun 1 à 3 atomes de carbone, ou un des deux radicaux $R_5$ et $R_6$ représente un groupe trifluorométhyle ou nitro, tandis que l'autre représente un atome d'hydrogène, ainsi que de leurs sels d'addition d'acide, caractérisé en ce qu'on fait réagir un sel d'un métal alcalin de l'indole de formule II:

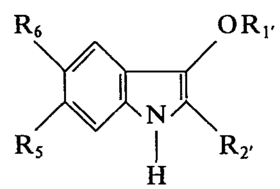

dans laquelle $R_5$ et $R_6$ ont les significations indiquées ci-dessus, $R_1'$ représente un groupe acétyle ou un groupe alkyle éventuellement substitué par un groupe phényle et $R_{2'}$ représente un groupe alcoxycarbonyle de la signification indiquée ci-dessus ou un groupe cyano, dans un solvant inerte

a) successivement avec un dihalogénoalcane de formule III:

$X_1 - A - X_2$

dans laquelle A a la signification indiquée ci-dessus, $X_1$ et $X_2$ peuvent être identiques ou différents et représentent chacun un atome d'halogène, et avec une amine de formule IV:

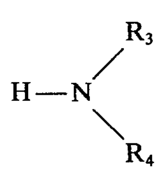

dans laquelle $R_3$ et $R_4$ ont les significations indiquées ci-dessus, ou uniquement avec une halogénalkylamine de formule V

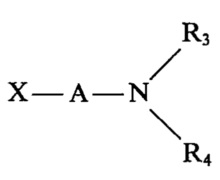

dans laquelle A a la signification indiquée ci-dessus, $R_3$ et $R_4$ ont les significations indiquées ci-dessus, hormis l'atome d'hydrogène, et X représente un atome d'halogène, pour obtenir des composés de formule I

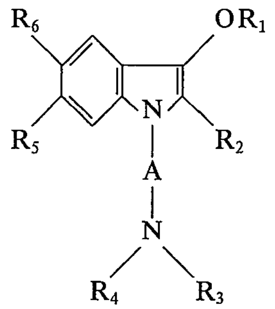

dans laquelle A, $R_3$, $R_4$, $R_5$ et $R_6$ ont les significations indiquées ci-dessus et $R_1$ représente un groupe acétyle ou un groupe alkyle éventuellement substitué par un groupe phényle et $R_2$ représente un groupe cyano ou alcoxycarbonyle,

b) si on le désire, on transforme un groupe cyano ou alcoxycarbonyle $R_2$ en groupe hydroxycarbonyle $R_2$,

c) si on le désire, on transforme un groupe cyano, alcoxycarbonyle ou hydroxycarbonyle $R_2$ en un groupe aminocarbonyle $R_2$ éventuellement mono- ou disubstitué,

d) si on le désire, dans les composés obtenus suivant a) ou c) et dans lesquels $R_1$ représente un groupe acétyle, on sépare le groupe acétyle $R_1$ par voie hydrolytique et on isole les composés 3-hydroxy,

e) si on le désire, dans les composés obtenus suivant a) ou c) et dans lesquels $R_1$ représente un groupe benzyle, on sépare le groupe benzyloxy par voie hydrogénolytique et on isole les composé 3-hydroxy,

f) si on le désire, dans les composés obtenus suivant a) ou c) et dans lesquels $R_1$ représente un groupe alkyle, on sépare le groupe alcoxy par voie hydrolytique et on isole les composés 3-hydroxy, et

g) on isole les bases ainsi obtenues et, si on le désire, on transforme celles-ci en sels d'addition d'acide correspondants.

13. Médicament constitué d'un ou de plusieurs composés suivant la revendication 1, ainsi que de substances auxiliaires et/ou de substances supports pharmaceutiques habituelles.